(19) **Europäisches Patentamt / European Patent Office / Office européen des brevets**

(11) **EP 3 971 201 B1**

(12) **EUROPEAN PATENT SPECIFICATION**

(45) Date of publication and mention
of the grant of the patent:
**28.01.2026 Bulletin 2026/05**

(21) Application number: **20805073.2**

(22) Date of filing: **15.05.2020**

(51) International Patent Classification (IPC):
**C12M 1/00** *(2006.01)*   **C12N 5/00** *(2006.01)*
**C07K 5/11** *(2006.01)*   **C07K 7/06** *(2006.01)*
**C08F 8/30** *(2006.01)*   **C08F 261/12** *(2006.01)*

(52) Cooperative Patent Classification (CPC):
(C-Sets available)
**C12N 5/0068; C08F 8/30; C08F 261/12;**
**C12M 23/20;** C07K 5/1019; C07K 7/06;
C12N 2533/30; C12N 2533/50        (Cont.)

(86) International application number:
**PCT/JP2020/019414**

(87) International publication number:
**WO 2020/230884 (19.11.2020 Gazette 2020/47)**

(54) **CELL CULTURING SCAFFOLD MATERIAL AND CELL CULTURING CONTAINER**

ZELLZÜCHTUNGSGERÜSTMATERIAL UND ZELLZÜCHTUNGSBEHÄLTER

MATÉRIAU D'ÉCHAFAUDAGE DE CULTURE CELLULAIRE ET RÉCIPIENT DE CULTURE CELLULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.05.2019 JP 2019092083**
**26.06.2019 JP 2019119079**

(43) Date of publication of application:
**23.03.2022 Bulletin 2022/12**

(73) Proprietor: **SEKISUI CHEMICAL CO., LTD.**
**Osaka-shi**
**Osaka**
**530-8565 (JP)**

(72) Inventors:
• **KOBAYASHI, Daigo**
**Mishima-gun, Osaka 618-0021 (JP)**
• **YUKAWA, Mayumi**
**Mishima-gun, Osaka 618-0021 (JP)**
• **TAKAKURA, Kenta**
**Mishima-gun, Osaka 618-0021 (JP)**
• **ISHII, Ryoma**
**Tokyo 105-8566 (JP)**

• **IGUCHI, Hiroki**
**Mishima-gun, Osaka 618-0021 (JP)**
• **ARAI, Yuuhei**
**Mishima-gun, Osaka 618-0021 (JP)**
• **HANEDA, Satoshi**
**Mishima-gun, Osaka 618-0021 (JP)**
• **INUI, Nobuhiko**
**Hasuda-shi, Saitama 349-0198 (JP)**

(74) Representative: **Ter Meer Steinmeister & Partner Patentanwälte mbB**
**Nymphenburger Straße 4**
**80335 München (DE)**

(56) References cited:
**JP-A- 2006 314 285      JP-A- 2015 070 832**
**JP-A- 2016 202 172      US-A1- 2012 322 145**
**US-A1- 2015 093 828      US-A1- 2016 304 839**

- **ZHU JUNMIN; TANG CHAD; KOTTKE-MARCHANT KANDICE; MARCHANT ROGER E: "Design and synthesis of biomimetic hydrogel scaffolds with controlled organization of cyclic RGD peptides", BIOCONJUGATE CHEMISTRY, vol. 20, no. 2, 2009, pages 333 - 339, XP009138216, ISSN: 1043-1802, DOI: 10.1021/bc800441v**
- **ALEXIS GOULET-HANSSENS, LAI WING SUN KAREN, KENNEDY TIMOTHY E., BARRETT CHRISTOPHER J.: "Photoreversible surfaces to regulate cell adhesion", BIOMACROMOLECULES, vol. 13, no. 9, 22 August 2012 (2012-08-22), pages 2958 - 2963, XP055760453, ISSN: 1525-7797, DOI: 10.1021/bm301037k**

Remarks:
The complete document including Reference Table(s) and the Sequence Listing(s) can be downloaded from the EPO website

(52) Cooperative Patent Classification (CPC): (Cont.)

C-Sets
**C08F 8/30, C08F 261/12;**
**C08F 261/12, C08F 220/06**

## Description

### TECHNICAL FIELD

The present invention relates to a cell culture scaffold material used for culturing cells. Also, the present invention relates to a cell culture vessel using the cell culture scaffold material.

### BACKGROUND ART

Cells of animals such as human, mouse, rat, pig, cow and monkey are used in research and development in academic fields, drug discovery fields, regenerative medicine fields, and the like. As a scaffold material used for culturing animal cells, adhesive proteins such as laminin and vitronectin, and natural polymer materials such as matrigel derived from mouse sarcoma are used. By using a natural polymer material as a scaffold material, a domain having a cell-adhesive amino acid sequence (such as Arg-Gly-Asp) of laminin, vitronectin or the like and an integrin on a cell surface are bound, the cells adhere well to the scaffold material, and the cells proliferate well.

Moreover, Patent Document 1 below discloses a protein containing a repeating structure and a cell adhesion sequence such as RGD sequence. This protein has a structure in which a specific Ala-rich site and a specific Ala-non-rich site are linked as the repeating structure. Further, Patent Document 1 describes that a material containing this protein can be used as a cell scaffold material.

Furthermore, scaffold materials using synthetic resins are also known as shown in Patent Documents 2 to 5 below.

Patent Document 2 below discloses a cell culture carrier composed of a molded product made of a polyvinyl acetal compound or a molded product made of the polyvinyl acetal compound and a water-soluble polysaccharide, the polyvinyl acetal compound having a degree of acetalization of 20 to 60 mol%.

In addition, Patent Document 3 below discloses a composition (scaffold material) containing a first fiber polymer scaffolding, in which the fibers of the first fiber polymer scaffolding are aligned. An aliphatic polyester and the like are used as the material of this fiber polymer.

Further, Patent Document 4 below discloses a cell culture method for maintaining an undifferentiated state of pluripotent stem cells, including culturing the pluripotent stem cells on an incubator having a surface coated with a polyrotaxan block copolymer.

Furthermore, Patent Document 5 below discloses a cell culture product comprising a substrate having a surface, a hydrophilic copolymer layer provided on the surface of the substrate, and a plurality of peptide chains bound to a surface of the hydrophilic copolymer layer, respectively. The hydrophilic copolymer layer is a layer copolymerized with a plurality of polyvinyl alcohol units, a plurality of polyvinyl alcohol derivative units, and a plurality of carboxylic acid group-containing units.

### Related Art Document

### Patent Document

> Patent Document 1: JP 2018-064542 A
> Patent Document 2: JP 2006-314285 A
> Patent Document 3: WO 2007/090102 A1
> Patent Document 4: JP 2017-023008 A
> Patent Document 5: JP 2015-070832 A

### SUMMARY OF THE INVENTION

### PROBLEMS TO BE SOLVED BY THE INVENTION

By using a natural polymer material as a scaffold material, cells after seeding proliferate well and pseudopodia extends well. However, natural polymer materials are expensive, have large variations between lots because they are naturally derived substances, or have safety concerns due to animal-derived components. Further, even when a polypeptide with a cell adhesion sequence such as RGD sequence as described in Patent Document 1 is used as a scaffold material, variation in cell adhesion and cell proliferation may occur depending on amino acid sequences other than the cell adhesion sequence.

On the other hand, the scaffold materials using synthetic resins as described in Patent Documents 2 to 5 are inexpensive, have less variation between lots and are excellent in safety, in comparison to scaffold materials using natural polymer materials. However, the conventional scaffold materials using synthetic resins as described in Patent Documents 2 to 4 have a problem that cell adhesion is low. Further, the scaffold material using the synthetic resin having a peptide chain described in Patent Document 5 can enhance cell adhesion to some extent, but may not be sufficient.

An object of the present invention is to provide a cell culture scaffold material having excellent cell adhesion. Also, an object of the present invention is to provide a cell culture vessel using the cell culture scaffold material.

## MEANS FOR SOLVING THE PROBLEMS

According to a broad aspect of the present invention, there is provided a cell culture scaffold material containing a peptide-conjugated polyvinyl acetal resin having a polyvinyl acetal resin portion and a peptide portion, in which the peptide portion has a cyclic peptide skeleton.

In a specific aspect of the cell culture scaffold material according to the present invention, the peptide portion has a cell-adhesive amino acid sequence.

In another specific aspect of the cell culture scaffold material according to the present invention, the cell-adhesive amino acid sequence has at least an RGD sequence, a YIGSR sequence, or a PDSGR sequence.

In still another specific aspect of the cell culture scaffold material according to the present invention, the cell-adhesive amino acid sequence has at least an RGD sequence represented by Formula (1) below:

Arg-Gly-Asp-X ...                Formula (1)

In Formula (1) above, X represents Gly, Ala, Val, Ser, Thr, Phe, Met, Pro, or Asn.

In still another specific aspect of the cell culture scaffold material according to the present invention, the cyclic peptide skeleton is composed of 4 or more and 10 or less amino acids.

In still another specific aspect of the cell culture scaffold material according to the present invention, the polyvinyl acetal resin portion and the peptide portion are bound via a linker portion.

In still another specific aspect of the cell culture scaffold material according to the present invention, the cell culture scaffold material has a sea-island structure.

According to a broad aspect of the present invention, there is provided a cell culture vessel including a vessel body and the above-mentioned cell culture scaffold material, in which the cell culture scaffold material is arranged on a surface of the vessel body.

## EFFECT OF THE INVENTION

According to the present invention, it is possible to provide a cell culture scaffold material and a cell culture vessel, having excellent cell adhesion.

## BRIEF DESCRIPTION OF DRAWINGS

[Fig. 1] Fig. 1 is a cross-sectional view schematically showing a cell culture vessel according to an embodiment of the present invention.
[Fig. 2] Figs. 2(a) and 2(b) are images showing the presence or absence of a sea-island structure.
[Fig. 3] Figs. 3(a), 3(b), and 3(c) are diagrams showing a relationship between SF and a planar shape of cells.

## MODES FOR CARRYING OUT THE INVENTION

Hereinafter, the details of the present invention will be described.

The cell culture scaffold material according to the present invention contains a peptide-conjugated polyvinyl acetal resin having a polyvinyl acetal resin portion and a peptide portion. In the cell culture scaffold material according to the present invention, the peptide portion has a cyclic peptide skeleton.

The cell culture scaffold material according to the present invention has the above constitution and thus has excellent cell adhesion. Further, the cell culture scaffold material according to the present invention has excellent extensibility of pseudopodia and excellent cell proliferation.

A cell culture scaffold material containing a peptide-conjugated polyvinyl acetal resin having a polyvinyl acetal resin portion and a peptide portion can enhance cell adhesion to some extent. However, when the peptide portion does not have a cyclic peptide skeleton, it may be difficult to enhance cell adhesion by using poorly adherent cells. On the other hand, in the cell culture scaffold material according to the present invention, since the peptide portion has a cyclic peptide skeleton, even the poorly adherent cells can enhance cell adhesion.

For example, in a cell culture scaffold material in which the peptide portion does not have a cyclic peptide skeleton, when aged mesenchymal stem cells that have been passaged for a long period of time, some iPS cell lines having low adhesion or the like are used, the cells may have difficulty adhering to scaffolding. On the other hand, in the cell culture scaffold material according to the present invention, since the peptide portion has a cyclic peptide skeleton, even such poorly adherent cells can enhance cell adhesion.

A mechanism according to (1) and (2) below is presumed as a mechanism for enhancing cell adhesion by the cell culture scaffold material according to the present invention, but the mechanism is not limited thereto.

(1) Due to a three-dimensional structure of the cyclic peptide skeleton, a specific amino acid sequence of the peptide portion (for example, a cell-adhesive amino acid sequence) is effectively exposed. Therefore, a specific amino acid sequence is easily recognized by the cell, and the peptide portion can stably bind to the cell.

(2) The peptide portion is not easily decomposed by a degrading enzyme produced by the cell, and the peptide portion is highly stable.

In the cell culture scaffold material according to the present invention, extension of pseudopodia like filamentous pseudopodia is observed in the cells after seeding, as in the case of using natural polymer materials such as matrigel. This extension of pseudopodia is hardly observed in conventional scaffold materials using synthetic resin materials.

In the cell culture scaffold material according to the present invention, cells adhere well to the cell culture scaffold material and the cells proliferate well even when seeding density of the cells is small.

Further, the cell culture scaffold material according to the present invention is inexpensive, has less variation between lots and is excellent in safety, in comparison to conventional cell scaffold materials using natural polymer. By using the cell scaffold material according to the present invention, load of cell quality control can be reduced.

(Cell culture scaffold material)

The cell culture scaffold material according to the present invention contains a peptide-conjugated polyvinyl acetal resin having a polyvinyl acetal resin portion and a peptide portion. As the peptide-conjugated polyvinyl acetal resin, only one type may be used, or two or more types may be used in combination.

The peptide-conjugated polyvinyl acetal resin has a polyvinyl acetal resin portion and a peptide portion. In the peptide-conjugated polyvinyl acetal resin, it is preferable that the polyvinyl acetal resin portion and the peptide portion are bound via a linker portion. Therefore, the peptide-conjugated polyvinyl acetal resin preferably has a polyvinyl acetal resin portion, a peptide portion, and a linker portion.

The peptide-conjugated polyvinyl acetal resin can be obtained, for example, by reacting a polyvinyl acetal resin with a linker and a peptide, as described later.

<Polyvinyl acetal resin portion>

The polyvinyl acetal resin is a compound derived from polyvinyl alcohol. As the polyvinyl acetal resin, only one type may be used, or two or more types may be used in combination.

The polyvinyl acetal resin portion preferably has an acetal group, a hydroxyl group, and an acetyl group in side chains. However, the polyvinyl acetal resin portion may not have, for example, an acetyl group. For example, by binding of all acetyl groups of the polyvinyl acetal resin to the linker, the polyvinyl acetal resin portion may not have an acetyl group.

The polyvinyl acetal resin can be synthesized by acetalizing polyvinyl alcohol with an aldehyde.

The aldehyde used for acetalizing polyvinyl alcohol is not particularly limited. Examples of the aldehyde include aldehydes having 1 to 10 carbon atoms. The aldehyde may or may not have a chain aliphatic group, a cyclic aliphatic group or an aromatic group. The aldehyde may be a chain aldehyde or a cyclic aldehyde.

Examples of the aldehyde include formaldehyde, acetaldehyde, propionaldehyde, butyraldehyde, pentanal, hexanal, heptanal, octanal, nonanal, decanal, acrolein, benzaldehyde, cinnamaldehyde, perillaldehyde, formylpyridine, formylimidazole, formylpyrrole, formylpiperidine, formyltriazole, formyltetrazole, formylindole, formylisoindole, formylpurine, formylbenzimidazole, formylbenzotriazole, formylquinoline, formylisoquinoline, formylquinoxaline, formylcinnoline, formylpteridine, formylfuran, formyloxolane, formyloxane, formylthiophene, formylthiolane, formylthiane, formyladenine, formylguanine, formylcytosine, formylthymine, formyluracil, and the like. As the aldehyde, only one type may be used, or two or more types may be used in combination.

The aldehyde is preferably formaldehyde, acetaldehyde, propionaldehyde, butyraldehyde, or pentanal, and more preferably butyraldehyde. Therefore, the polyvinyl acetal resin is more preferably a polyvinyl butyral resin, the polyvinyl acetal resin portion is more preferably a polyvinyl butyral resin portion, and the peptide-conjugated polyvinyl alcohol derivative is more preferably a peptide-conjugated polyvinyl butyral resin.

The blending amount of the aldehyde can be appropriately set according to the intended amount of acetal group. From the viewpoint of increasing efficiency of an acetalization reaction and easily removing unreacted aldehyde, the amount of the aldehyde added is preferably 60 mol% or more and more preferably 65 mol% or more, and is preferably 95 mol% or less and more preferably 90 mol% or less, based on 100 mol% of polyvinyl alcohol.

Average degree of polymerization of the polyvinyl acetal resin portion and the polyvinyl acetal resin is preferably 100 or more, more preferably 200 or more, further preferably 500 or more, and particularly preferably 1500 or more, and is preferably 6000 or less, more preferably 3000 or less, and further preferably 2500 or less. When the average degree of polymerization is the above lower limit or more, swelling due to liquid medium can be effectively suppressed, so that strength of the cell culture scaffold material can be maintained satisfactorily. Therefore, cell proliferation can be enhanced. Further, when the average degree of polymerization is the above upper limit or less, handleability can be improved, and moldability of the cell culture scaffold material can be improved. The average degree of polymerization of the polyvinyl acetal resin portion and the polyvinyl acetal resin is usually the same as average degree of polymerization of the raw material polyvinyl alcohol, thus can be determined by the average degree of polymerization of polyvinyl alcohol.

Number average molecular weights (Mn) of the polyvinyl acetal resin portion and the polyvinyl acetal resin are preferably 10,000 or more and preferably 600,000 or less. Weight average molecular weights (Mw) of the polyvinyl acetal resin portion and the polyvinyl acetal resin are preferably 2,000 or more and preferably 1,200,000 or less. Also, in the polyvinyl acetal resin portion and the polyvinyl acetal resin, a ratio (Mw/Mn) of the weight average molecular weight (Mw) to the number average molecular weight (Mn) is preferably 2.0 or more and preferably 40 or less. When the Mn, the Mw and the Mw/Mn are the above lower limit or more and the above upper limit or less, the strength of the cell culture scaffold material can be increased.

The number average molecular weights (Mn) and weight average molecular weights (Mw) of the polyvinyl acetal resin portion and the polyvinyl acetal resin can be obtained as polystyrene equivalent values, for example, by gel permeation chromatography (GPC) analysis using tetrahydrofuran (THF) as a solvent.

Degrees of acetalization (degree of butyralization in the case of polyvinyl butyral resin) of the polyvinyl acetal resin portion and the polyvinyl acetal resin are preferably 40 mol% or more and more preferably 50 mol% or more, and are 90 mol% or less and more preferably 85 mol% or less. When the degree of acetalization is the above lower limit or more, fixation of the cells can be further enhanced, and the cells proliferate efficiently. When the degree of acetalization is the above upper limit or less, solubility in a solvent can be improved.

Hydroxyl group contents (amounts of hydroxyl groups) of the polyvinyl acetal resin portion and the polyvinyl acetal resin are preferably 15 mol% or more and more preferably 20 mol% or more, and are preferably 45 mol% or less, more preferably 30 mol% or less, and more preferably 25 mol% or less.

Degrees of acetylation (amounts of acetylation groups) of the polyvinyl acetal resin portion and the polyvinyl acetal resin are preferably 1 mol% or more and more preferably 2 mol% or more, and are preferably 5 mol% or less and more preferably 4 mol% or less. When the degree of acetylation is the above lower limit or more and the above upper limit or less, a reaction efficiency between the polyvinyl acetal resin and a linker can be enhanced.

The degree of acetalization, the degree of acetylation, and the amount of hydroxyl groups of the polyvinyl acetal resin portion and the polyvinyl acetal resin can be measured by $^1$H-NMR (nuclear magnetic resonance spectrum).

<Peptide portion>

The peptide portion is a structural part derived from the peptide in the peptide-conjugated polyvinyl acetal resin

The peptide portion has an amino acid sequence.

The peptide constituting the peptide portion may be an oligopeptide or a polypeptide. As the peptide, only one type may be used, or two or more types may be used in combination.

The peptide portion has a cyclic peptide skeleton. The cyclic peptide skeleton is a cyclic skeleton composed of a plurality of amino acids. The peptide portion may have only a cyclic peptide skeleton, or may have a cyclic peptide skeleton and a skeleton (such as a chain peptide skeleton) other than the cyclic peptide skeleton.

The peptide portion is preferably composed of 4 or more amino acids and more preferably composed of 5 or more amino acids, and is preferably composed of 15 or less amino acids and more preferably composed of 10 or less amino acids. When the number of amino acids constituting the peptide portion is the above lower limit or more and the above upper limit or less, recognition of a specific amino acid sequence by cells can be enhanced, and cell adhesion and proliferation can be further enhanced. Further, when the number of amino acids constituting the peptide portion is the above lower limit or more and the above upper limit or less, extensibility of pseudopodia can be further improved.

The cyclic peptide skeleton is preferably composed of 4 or more amino acids and more preferably composed of 5 or more amino acids, and is preferably composed of 10 or less amino acids and more preferably composed of 7 or less amino acids. When the number of amino acids constituting the cyclic peptide skeleton is the above lower limit or more, the recognition of a specific amino acid sequence by cells can be enhanced, and cell adhesion and proliferation can be further enhanced. Further, when the number of amino acids constituting the cyclic peptide skeleton is the above lower limit or more and the above upper limit or less, the extensibility of pseudopodia can be further improved. From the viewpoint of keeping cost low, the number of amino acids constituting the cyclic peptide skeleton is preferably 5.

The peptide portion preferably has a cell-adhesive amino acid sequence. The cell-adhesive amino acid sequence refers to an amino acid sequence whose cell adhesion activity has been confirmed by phage display method, sepharose beads method, or plate coating method. As the phage display method, for example, a method described in "The Journal of Cell Biology, Volume 130, Number 5, September 1995 1189-1196" can be used. As the sepharose beads method, for example, a method described in "Protein, Nucleic Acid and Enzyme, Vol.45 No.15 (2000) 2477" can be used. As the plate coating method, for example, a method described in "Protein, Nucleic Acid and Enzyme, Vol.45 No.15 (2000) 2477" can be used.

Examples of the cell-adhesive amino acid sequence include RGD sequence (Arg-Gly-Asp), YIGSR sequence (Tyr-Ile-Gly-Ser-Arg), PDSGR sequence (Pro-Asp-Ser-Gly-Arg), HAV sequence (His-Ala-Val), ADT sequence (Ala-Asp-Thr), QAV sequence (Gln-Ala-Val), LDV sequence (Leu-Asp-Val), IDS sequence (Ile-Asp-Ser), REDV sequence (Arg-Glu-Asp-Val), IDAPS sequence (Ile-Asp-Ala-Pro-Ser), KQAGDV sequence (Lys-Gln-Ala-Gly-Asp-Val), TDE sequence (Thr-Asp-Glu), and the like. In addition, examples of the cell-adhesive amino acid sequence include sequences described in "Medicina Philosophica, Vol. 9, No. 7, pp. 527-535, 1990" and "Journal of Osaka Women's and Children's Hospital, Vol. 8, No. 1, pp. 58-66, 1992", and the like. The peptide portion may have only one type of cell-adhesive amino acid sequence, or may have two or more types.

The cell-adhesive amino acid sequence preferably has at least one of the above-mentioned cell-adhesive amino acid sequences, more preferably has at least an RGD sequence, a YIGSR sequence or a PDSGR sequence, and further preferably has at least an RGD sequence represented by the following formula (1). In this case, cell adhesion and proliferation can be further enhanced, and the extensibility of pseudopodia can be further improved.

Arg-Gly-Asp-X ...　　　　　Formula (1)

In Formula (1) above, X represents Gly, Ala, Val, Ser, Thr, Phe, Met, Pro, or Asn.

When the peptide portion has the cell-adhesive amino acid sequence, the cyclic peptide skeleton may or may not have the cell-adhesive amino acid sequence. From the viewpoint of enhancing the recognition of the cell-adhesive amino acid sequence by cells and further enhancing cell adhesion and proliferation, it is preferable that the cyclic peptide skeleton has the cell-adhesive amino acid sequence.

When the peptide portion has the cell-adhesive amino acid sequence, an amino acid at an N-terminal or an amino acid at a C-terminal of the cell-adhesive amino acid sequence and a linker may be bound, and an amino acid constituting an amino acid sequence of a portion different from the cell-adhesive amino acid sequence and a linker may be bound.

When the peptide portion has the cell-adhesive amino acid sequence, it is more preferable that the amino acid constituting the amino acid sequence of a portion different from the cell-adhesive amino acid sequence and a linker portion are bound. In this case, cell adhesion and proliferation can be further enhanced, and the extensibility of pseudopodia can be further improved.

The content of the peptide portion in 100% by weight of the peptide-conjugated polyvinyl acetal resin is preferably 0.01% by weight or more, more preferably 0.1% by weight or more, further preferably 1% by weight or more, and particularly preferably 5% by weight or more. The content of the peptide portion in 100% by weight of the peptide-conjugated polyvinyl acetal resin is preferably 30% by weight or less, more preferably 25% by weight or less, further preferably 20% by weight or less, and particularly preferably 15% by weight or less. When the content of the peptide portion is the above lower limit or more, cell adhesion and proliferation can be even more enhanced, and the extensibility of pseudopodia can be even more improved.

In the peptide-conjugated polyvinyl acetal resin, the content of the peptide portion is preferably 0.01 mol% or more, more preferably 0.1 mol% or more, even more preferably 1 mol% or more, further preferably 5 mol% or more, and particularly preferably 10 mol% or more. In the peptide-conjugated polyvinyl acetal resin, the content of the peptide portion is preferably 60 mol% or less, more preferably 50 mol% or less, further preferably 35 mol% or less, and particularly preferably 25 mol% or less. When the content of the peptide portion is the above lower limit or more, cell adhesion and proliferation can be even more enhanced, and the extensibility of pseudopodia can be even more improved. When the content of the peptide portion is the above upper limit or less, production cost can be suppressed. The content (mol%) of the peptide portion is amount of substance of the peptide portion with respect to the total of the amount of substance of structural units constituting the peptide-conjugated polyvinyl acetal resin

In the peptide-conjugated polyvinyl acetal resin, a molar ratio of the content of the peptide portion to a total content of the acetal group, the hydroxyl group and the acetyl group (content of the peptide portion/total content of the acetal group, the hydroxyl group and the acetyl group) is preferably 0.0001 or more, and more preferably 0.001 or more. When the molar ratio (content of the peptide portion/total content of the acetal group, the hydroxyl group and the acetyl group) is the above lower limit or more, cell adhesion and proliferation can be even more enhanced, and the extensibility of pseudopodia can be even more improved. In the peptide-conjugated polyvinyl acetal resin, an upper limit of the molar ratio of the content of the peptide portion to the total content of the acetal group, the hydroxyl group and the acetyl group (content of the peptide portion/total content of the acetal group, the hydroxyl group and the acetyl group) is not particularly limited. From the viewpoint of production cost and the like, the molar ratio (content of the peptide portion/total content of the acetal group, the hydroxyl group and the acetyl group) is preferably 0.2 or less.

The content of the peptide portion can be measured by FT-IR or LC-MS.

<Linker portion>

The linker portion is a structural part derived from the linker in the peptide-conjugated polyvinyl acetal resin

The linker portion is located between the polyvinyl acetal resin portion and the peptide portion. The polyvinyl acetal resin portion and the peptide portion are bound via the linker portion. The linker portion is formed by a linker (crosslinking agent).

As the linker, only one type may be used, or two or more types may be used in combination.

The linker is preferably a compound having a functional group capable of condensing with the carboxyl group or amino group of the peptide. Examples of the functional group capable of condensing with the carboxyl group or amino group of the peptide include a carboxyl group, a thiol group, an amino group, and the like. From the viewpoint of well reacting with a peptide, the linker is preferably a compound having a carboxyl group.

Examples of the linker having a carboxyl group include (meth)acrylic acid, a carboxyl group-containing acrylamide, and the like. By using a carboxylic acid having a polymerizable unsaturated group (carboxylic acid monomer) as the linker having a carboxyl group, the carboxylic acid monomer can be polymerized by graft polymerization at the time of introduction of the linker, so that the number of the carboxyl groups capable of reacting with a peptide can be increased.

From the viewpoint of satisfactorily binding a polyvinyl acetal resin and a peptide, the linker is preferably (meth) acrylic acid and more preferably acrylic acid.

The peptide-conjugated polyvinyl acetal resin can be synthesized, for example, as follows.

(1) A polyvinyl acetal resin is reacted with a linker to obtain a reactant in which the polyvinyl acetal resin and the linker are bound. (2) The obtained reactant is reacted with a peptide to obtain a peptide-conjugated polyvinyl acetal resin

In (1) above, examples of a method for obtaining a reactant in which the polyvinyl acetal resin and the linker are bound includes a method of acetalizing a copolymer of polyvinyl alcohol and a carboxylic acid having a polymerizable unsaturated group, a method of graft-copolymerizing a polyvinyl acetal resin and a linker (for example, a carboxylic acid monomer) under ultraviolet irradiation, and the like. The method for obtaining a reactant is preferably the graft copolymerization method. In this case, since the carboxylic acid monomer can be polymerized by graft polymerization, the number of carboxyl groups capable of reacting with a peptide can be increased.

In (2) above, a peptide-conjugated polyvinyl acetal resin having a polyvinyl acetal resin portion, a peptide portion and a linker portion can be obtained by dehydration-condensing a carboxyl group derived from the linker in the obtained reactant and an amino group of the peptide.

In the peptide-conjugated polyvinyl acetal resin, the carboxyl group derived from the linker may or may not remain. The content of the carboxyl groups of the peptide-conjugated polyvinyl acetal resin is preferably 0.1 mol% or more, more preferably 0.5 mol% or more, preferably 2 mol% or less, and more preferably 1.5 mol% or less. When the content of the carboxyl groups is the above lower limit or more and the above upper limit or less, cell adhesion and proliferation can be even more enhanced, and the extensibility of pseudopodia can be even more improved. The content (mol%) of the carboxyl groups is amount of substance of the carboxyl group with respect to the total of the amount of substance of structural units constituting the peptide-conjugated polyvinyl acetal resin

From the viewpoint of further enhancing cell adhesion and proliferation, the cell culture scaffold material preferably has a phase-separated structure. The phase-separated structure has at least a first phase and a second phase.

Examples of the phase-separated structure include microphase-separated structures such as a sea-island structure, a cylinder structure, a gyroid structure, and a lamellar structure. In the sea-island structure, for example, the first phase can be a sea part and the second phase can be an island part. In the cylinder structure, gyroid structure, or lamellar structure, for example, a phase having a largest surface area can be the first phase, and a phase having a second largest surface area can be the second phase. The cell culture scaffold material has a continuous phase and a discontinuous phase, thereby enhancing affinity with cells, and cell adhesion and proliferation can be further enhanced.

The phase-separated structure is preferably a sea-island structure. The cell culture scaffold material preferably has a sea-island structure. In this case, cell adhesion and proliferation can be further enhanced.

When the cell culture scaffold material has a sea-island structure, surface area fraction of the island part (second phase) with respect to the entire surface of the cell culture scaffold material is preferably 0.01 or more, more preferably 0.1 or more, further preferably 0.2 or more, preferably 0.95 or less, more preferably 0.9 or less, and further preferably 0.8 or less. When the surface area fraction is the above lower limit or more and the above upper limit or less, cell adhesion can be further enhanced.

When the cell culture scaffold material has a sea-island structure, it is preferable that the island part contains a peptide portion. That is, it is preferable that the cell culture scaffold material has a sea part and an island part, and the island part contains a peptide portion. In this case, adhesion domains of the cells are accumulated in the island part, whereby cell adhesion can be further enhanced.

The presence or absence of a phase-separated structure can be confirmed by, for example, an atomic force microscope (AFM), a transmission electron microscope (TEM), a scanning electron microscope (SEM), or the like. Further, the surface area fraction can be obtained from a microscope observation image using image analysis software such as ImageJ.

The phase-separated structure can be formed, for example, by increasing the content of peptide portion and forming a phase-separated structure between or within molecules of a peptide-conjugated polyvinyl acetal resin

From the viewpoint of effectively exerting the effect of the present invention and enhancing productivity, the content of the peptide-conjugated polyvinyl acetal resin in 100% by weight of the cell culture scaffold material is preferably 90% by weight or more, more preferably 95% by weight or more, further preferably 97.5% by weight or more, particularly preferably 99% by weight or more, and most preferably 100% by weight (whole amount). Therefore, it is most preferable that the cell culture scaffold material is the peptide-conjugated polyvinyl acetal resin.

When the content of the peptide-conjugated polyvinyl acetal resin is the above lower limit or more, the effect of the present invention can be even more effectively exhibited.

The cell culture scaffold material may contain a polymer other than the peptide-conjugated polyvinyl acetal resin

Examples of the polymer include polyolefin resins, polyether resins, polyvinyl alcohol resins, polyesters, epoxy resins, polyamide resins, polyimide resins, polyurethane resins, polycarbonate resins, celluloses, polypeptides, and the like. As the polymer, only one type may be used, or two or more types may be used in combination.

From the viewpoint of effectively exerting the effect of the present invention, the smaller content of the polymer other than the peptide-conjugated polyvinyl acetal resin, the better. The content of the polymer in 100% by weight of the cell culture scaffold material is preferably 10% by weight or less, more preferably 5% by weight or less, further preferably 2.5% by weight or less, particularly preferably 1% by weight or less, and most preferably 0% by weight (not contained). Therefore, it is most preferable that the cell culture scaffold material does not contain a polymer other than the peptide-conjugated polyvinyl acetal resin

It is preferable that the cell culture scaffold material according to the present invention does not substantially contain animal-derived raw materials. By substantially not containing animal-derived raw materials, it is possible to provide a cell culture scaffold material that has less variation between lots and is excellent in cost and safety. In addition, the phrase "does not substantially contain animal-derived raw materials" means that the animal-derived raw materials in the cell culture scaffold material are 3% by weight or less. In the cell culture scaffold material according to the present invention, the animal-derived raw materials in the cell culture scaffold material are preferably 1% by weight or less, and more preferably 0% by weight. That is, it is more preferable that the cell culture scaffold material does not contain animal-derived raw materials in the cell culture scaffold material.

The cell culture scaffold material may be prepared on a surface of a vessel body described later. For example, the peptide-conjugated polyvinyl acetal resin may be obtained by coating a synthetic resin having a polyvinyl acetal resin portion and a linker on the surface of the vessel body to form a resin film, and reacting the synthetic resin and a peptide on a surface of the resin film.

(Other details of cell culture scaffold material)

The cell culture scaffold material according to the present invention is used for culturing cells. The cell culture scaffold material according to the present invention is used as a scaffold for cells when culturing the cells.

Examples of the cells include cells of animals such as human, mouse, rat, pig, cow and monkey. In addition, examples of the cells include somatic cells and the like, and examples thereof include stem cells, progenitor cells, mature cells, and the like. The somatic cells may be cancer cells.

Examples of the mature cells include nerve cells, cardiomyocytes, retinal cells, hepatocytes, and the like.

Examples of the stem cells include mesenchymal stem cells (MSCs), iPS cells, ES cells, Muse cells, embryonic cancer cells, embryonic germ cells, mGS cells, and the like.

Shape of the cell culture scaffold material is not particularly limited. The cell culture scaffold material may be in a film form, a particle form, a fibrous form, or a porous body form. The film form includes a film form and a sheet form.

The cell culture scaffold material is preferably used for two-dimensional culture (plane culture), three-dimensional culture or suspension culture of cells, and more preferably used for two-dimensional culture (plane culture).

In addition, the cell culture scaffold material can also be used as a cell culture carrier (medium) containing the cell culture scaffold material and polysaccharides. The polysaccharide is not particularly limited, and a conventionally known polysaccharide can be used. The polysaccharide is preferably a water-soluble polysaccharide.

Further, the cell culture scaffold material can also be used as a fiber for cell culture having a fiber body and a cell culture scaffold material arranged on the surface of the fiber body. In this case, the cell culture scaffold material is preferably coated on the surface of the fiber body, and is preferably a coated material. In this fiber for cell culture, a cell culture scaffold material may be present in the fiber body. For example, the cell culture scaffold material can be present in the fiber body by impregnating or kneading the fiber body into the liquid cell culture scaffold material. In general, stem cells have a property of being difficult to adhere to a planar structure and easily adhering to a three-dimensional structure such as a fibrous structure. Therefore, a fiber for cell culture is suitably used for three-dimensional culture of stem cells. Among stem cells, it is more preferably used for three-dimensional culture of adipose stem cells.

The synthetic resin in the cell culture scaffold material may be crosslinked. The cell culture scaffold material containing a crosslinked synthetic resin is effectively suppressed in water swelling properties and can increase strength. By using a crosslinking agent, the synthetic resin can be crosslinked.

(Cell culture vessel)

The cell culture vessel according to the present invention includes a vessel body and the above-mentioned cell culture scaffold material, and the cell culture scaffold material is arranged on a surface of the vessel body. The cell culture vessel includes the cell culture scaffold material in at least a part of cell culture area.

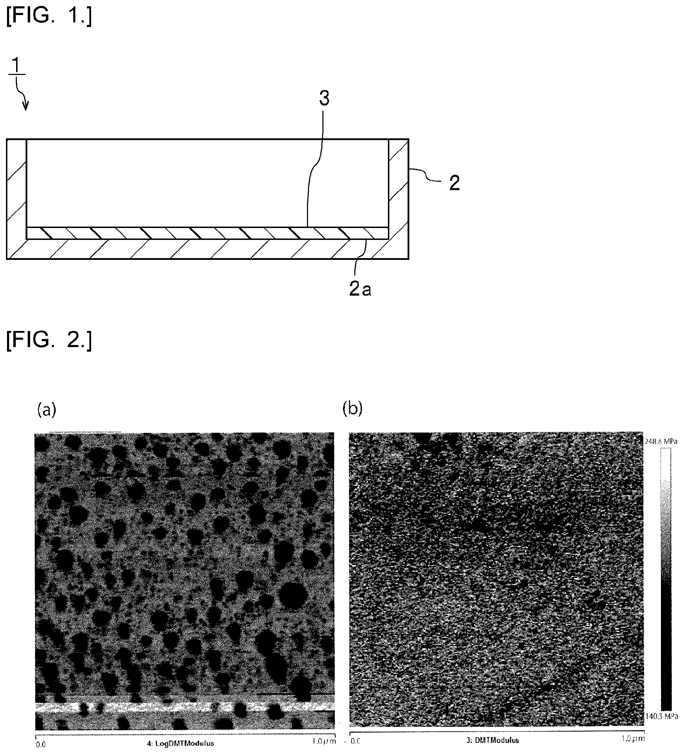

Fig. 1 is a cross-sectional view schematically showing a cell culture vessel according to an embodiment of the present invention.

A cell culture vessel 1 includes a vessel body 2 and a cell culture scaffold material 3. The cell culture scaffold material 3 is arranged on a surface 2a of the vessel body 2. The cell culture scaffold material 3 is arranged on a bottom surface of the vessel body 2. Cells can be cultured in plane by adding a liquid medium to the cell culture vessel 1 and seeding cells such as cell mass on a surface of the cell culture scaffold material 3.

The vessel body may include a first vessel body, and a second vessel body such as a cover glass on the bottom surface of the first vessel body. The first vessel body and the second vessel body may be separable. In this case, the cell culture scaffold material may be arranged on the surface of the second vessel body.

As the vessel body, a conventionally known vessel body (vessel) can be used. Shape and size of the vessel body are not particularly limited.

Examples of the vessel body include a cell culture plate provided with one or a plurality of wells (holes), a cell culture flask, and the like. The number of wells in the plate is not particularly limited. The number of wells is not particularly limited, and examples thereof include 2, 4, 6, 12, 24, 48, 96, 384, and the like. Shape of the well is not particularly limited, and examples thereof include a perfect circle, an ellipse, a triangle, a square, a rectangle, a pentagon, and the like. Shape of the bottom surface of the well is not particularly limited, and examples thereof include a flat bottom, a round bottom, unevenness, and the like.

Material of the vessel body is not particularly limited, and examples thereof include resins, metals, and inorganic materials. Examples of the resin include polystyrene, polyethylene, polypropylene, polycarbonate, polyester, polyisoprene, cycloolefin polymer, polyimide, polyamide, polyamideimide, (meth)acrylic resin, epoxy resin, silicone, and the like. Examples of the metal include stainless steel, copper, iron, nickel, aluminum, titanium, gold, silver, platinum, and the like. Examples of the inorganic material include silicon oxide (glass), aluminum oxide, titanium oxide, zirconium oxide, iron oxide, silicon nitride, and the like.

The present invention will be described in more detail below with reference to Examples and Comparative Examples. The present invention is not limited to these examples.

The content of structural units in the obtained synthetic resin was measured by $^1$H-NMR (nuclear magnetic resonance spectrum) after dissolving a synthetic resin in DMSO-d6 (dimethylsulfoxide). Also, the content of peptide in the peptide-conjugated polyvinyl alcohol derivative was measured by FT-IR or LC-MS. Tables 1 to 3 show degrees of acetalization (degrees of butyralization), amounts of hydroxyl groups, degrees of acetylation, contents of carboxyl groups, and contents of peptide portion of the obtained synthetic resins.

(Example 1)

Preparation of polyvinyl acetal resin (PVB1):

A reactor equipped with a stirrer was charged with 2700 mL of ion-exchanged water, 300 parts by weight of polyvinyl alcohol with an average degree of polymerization of 1700 and a degree of saponification of 99 mol%, followed by dissolution by heating with stirring to obtain a solution. To the obtained solution, 35% by weight hydrochloric acid as a catalyst was added such that the concentration of hydrochloric acid became 0.2% by weight. Subsequently, temperature was adjusted to 15°C, and 22 parts by weight of n-butyraldehyde was added thereto with stirring. Then, 148 parts by weight of n-butyraldehyde was added thereto to precipitate a white particulate polyvinyl acetal resin (polyvinyl butyral resin). Fifteen minutes after the precipitation, 35% by weight hydrochloric acid was added such that the concentration of hydrochloric acid became 1.8% by weight, and then the mixture was heated to 50°C and kept at 50°C for 2 hours. Next, the solution was cooled and neutralized, and then the polyvinyl butyral resin was washed with water and dried to obtain a polyvinyl acetal resin (polyvinyl butyral resin (PVB1), an average degree of polymerization of 1700, a degree of acetalization (degree of butyralization) of 70 mol%, an amount of hydroxyl groups of 27 mol%, and a degree of acetylation of 3 mol%).

Introduction of linker:

Nighty nine parts by weight of the obtained polyvinyl acetal resin and 1 part by weight of acrylic acid (linker) were dissolved in 300 parts by weight of THF and reacted in the presence of a photoradical polymerization initiator for 20 minutes under ultraviolet irradiation to graft-copolymerize a polyvinyl acetal resin with acrylic acid, thereby introducing the linker. One part by weight of the polyvinyl acetal resin into which the linker was introduced was dissolved in 19 parts by weight of butanol. The obtained solution (150 μL) was discharged onto a surface of a φ22 mm cover glass ("22 round No. 1" manufactured by Matsunami Glass Ind., Ltd.) subjected to dust removal with an air duster, rotated at 2000 rpm for 20 seconds using a spin coater, and then heated at 60°C for 60 minutes to obtain a resin film with a smooth surface.

Formation of peptide portion:

A cyclic peptide having an amino acid sequence of Arg-Gly-Asp-Phe-Lys (five amino acid residues, a cyclic skeleton formed by binding Arg and Lys, D-form Phe, described as c-RGDfK in the table) was prepared. One part by weight of this peptide and 1 part by weight of 1-ethyl-3-(3-dimethylaminopropyl)carbodiimide hydrochloride (condensing agent) were added to phosphate buffered saline containing neither calcium nor magnesium so that the final concentration of the peptide is 1 mM to prepare a peptide-containing solution. One part by weight of this peptide-containing liquid was added to a spin-coated resin film (polyvinyl acetal resin with a linker formed) and reacted to dehydrate and condense a carboxyl group of the linker and an amino group of Lys of the peptide. In this way, a peptide-conjugated polyvinyl acetal resin having a polyvinyl acetal resin portion, a linker portion and a peptide portion was prepared.

The obtained peptide-conjugated polyvinyl acetal resin had a degree of acetalization (degree of butyralization) of 69.3 mol%, an amount of hydroxyl groups of 26.7 mol%, a degree of acetylation of 3.0 mol%, a content of carboxyl groups of 0.9 mol%, and a content of peptide portion of 0.1 mol%.

Preparation of cell culture vessel:

A laminate of the obtained peptide-conjugated polyvinyl acetal resin and the cover glass was arranged on a φ22 mm polystyrene dish to obtain a cell culture vessel.

(Example 2)

Preparation of polyvinyl acetal resin (PVB2):

A reactor equipped with a stirrer was charged with 2700 mL of ion-exchanged water, 300 parts by weight of polyvinyl alcohol with an average degree of polymerization of 1700 and a degree of saponification of 99 mol%, followed by dissolution by heating with stirring to obtain a solution. To the obtained solution, 35% by weight hydrochloric acid as a catalyst was added such that the concentration of hydrochloric acid became 0.2% by weight. Subsequently, temperature was adjusted to 15°C, and 22 parts by weight of n-butyraldehyde was added thereto with stirring. Then, 143 parts by weight of n-butyraldehyde was added to precipitate a white particulate polyvinyl acetal resin (polyvinyl butyral resin). Fifteen minutes after the precipitation, 35% by weight hydrochloric acid was added such that the concentration of hydrochloric acid became 1.8% by weight, and then the mixture was heated to 50°C and kept at 50°C for 2 hours. Next, the solution was cooled and neutralized, and then the polyvinyl butyral resin was washed with water and dried to obtain a polyvinyl acetal resin (polyvinyl butyral resin (PVB2), an average degree of polymerization of 1700, a degree of acetalization (degree of butyralization) of 69 mol%, an amount of hydroxyl groups of 28 mol%, and a degree of acetylation of 3 mol%).

Introduction of linker:

Seventy parts by weight of the obtained polyvinyl acetal resin and 30 parts by weight of acrylic acid (linker) were dissolved in 300 parts by weight of THF and reacted in the presence of a photoradical polymerization initiator for 20 minutes under ultraviolet irradiation to graft-copolymerize a polyvinyl acetal resin with acrylic acid, thereby introducing the linker. One part by weight of the polyvinyl acetal resin into which the linker was introduced was dissolved in 19 parts by weight of butanol. The obtained solution (150 μL) was discharged onto a surface of a φ22 mm cover glass ("22 round No. 1" manufactured by Matsunami Glass Ind., Ltd.) subjected to dust removal with an air duster, rotated at 2000 rpm for 20 seconds using a spin coater, and then heated at 60°C for 60 minutes to obtain a resin film with a smooth surface.

Formation of peptide portion:

A peptide-conjugated polyvinyl acetal resin was prepared in the same manner as in Example 1 except that the obtained resin film (polyvinyl acetal resin into which a linker was introduced) was used and the amount of peptide added was 30 parts by weight.

Preparation of cell culture vessel:

A cell culture vessel was obtained in the same manner as in Example 1.

(Example 3)

Preparation of polyvinyl acetal resin (PVB3):

A reactor equipped with a stirrer was charged with 2700 mL of ion-exchanged water, 300 parts by weight of polyvinyl alcohol with an average degree of polymerization of 1700 and a degree of saponification of 99 mol%, followed by dissolution by heating with stirring to obtain a solution. To the obtained solution, 35% by weight hydrochloric acid as a catalyst was added such that the concentration of hydrochloric acid became 0.2% by weight. Subsequently, temperature was adjusted to 15°C, and 22 parts by weight of n-butyraldehyde was added thereto with stirring. Then, 133 parts by weight of n-butyraldehyde was added to precipitate a white particulate polyvinyl acetal resin (polyvinyl butyral resin). Fifteen minutes after the precipitation, 35% by weight hydrochloric acid was added such that the concentration of hydrochloric acid became 1.8% by weight, and then the mixture was heated to 50°C and kept at 50°C for 2 hours. Next, the solution was cooled and neutralized, and then the polyvinyl butyral resin was washed with water and dried to obtain a polyvinyl acetal resin (polyvinyl butyral resin (PVB3), an average degree of polymerization of 1700, a degree of acetalization (degree of butyralization) of 63 mol%, an amount of hydroxyl groups of 34 mol%, and a degree of acetylation of 3 mol%).

Introduction of linker:

A linker was introduced in the same manner as in Example 2 except that the obtained polyvinyl acetal resin was used. Further, a resin film having a smooth surface was obtained in the same manner as in Example 2.

Formation of peptide portion:

A peptide-conjugated polyvinyl acetal resin was prepared in the same manner as in Example 2 except that the obtained resin film (polyvinyl acetal resin into which a linker was introduced) was used.

Preparation of cell culture vessel:

A cell culture vessel was obtained in the same manner as in Example 1.

(Example 4)

Preparation of polyvinyl acetal resin (PVB4):

A reactor equipped with a stirrer was charged with 2700 mL of ion-exchanged water, 300 parts by weight of polyvinyl alcohol with an average degree of polymerization of 1700 and a degree of saponification of 99 mol%, followed by dissolution by heating with stirring to obtain a solution. To the obtained solution, 35% by weight hydrochloric acid as a catalyst was added such that the concentration of hydrochloric acid became 0.2% by weight. Subsequently, temperature was adjusted to 15°C, and 22 parts by weight of n-butyraldehyde was added thereto with stirring. Then, 133 parts by weight of n-butyraldehyde was added to precipitate a white particulate polyvinyl acetal resin (polyvinyl butyral resin). Fifteen minutes after the precipitation, 35% by weight hydrochloric acid was added such that the concentration of hydrochloric acid became 1.8% by weight, and then the mixture was heated to 50°C and kept at 50°C for 2 hours. Next, the solution was cooled and neutralized, and then the polyvinyl butyral resin was washed with water and dried to obtain a polyvinyl acetal resin (polyvinyl butyral resin (PVB4), an average degree of polymerization of 1700, a degree of acetalization (degree of butyralization) of 50 mol%, an amount of hydroxyl groups of 47 mol%, and a degree of acetylation of 3 mol%).

Introduction of linker:

A linker was introduced in the same manner as in Example 2 except that the obtained polyvinyl acetal resin was used. Further, a resin film having a smooth surface was obtained in the same manner as in Example 2.

Formation of peptide portion:

A peptide-conjugated polyvinyl acetal resin was prepared in the same manner as in Example 2 except that the obtained resin film (polyvinyl acetal resin into which a linker was introduced) was used.

Preparation of cell culture vessel:

A cell culture vessel was obtained in the same manner as in Example 1.

(Comparative Example 1)

A peptide-conjugated polyvinyl acetal resin and a cell culture vessel were prepared in the same manner as in Example 1 except that a linear peptide having an amino acid sequence of Arg-Gly-Asp-Ser (four amino acid residues, described as RGDS in the table) was used, and the carboxyl group of the linker and the amino group of Arg of the peptide were dehydrated and condensed.

(Comparative Example 2)

A peptide-conjugated polyvinyl acetal resin and a cell culture vessel were prepared in the same manner as in Example 2 except that a linear peptide having an amino acid sequence of Gly-Arg-Gly-Asp-Ser (five amino acid residues, described as GRGDS in the table) was used, and the carboxyl group of the linker and the amino group of Gly of the peptide were dehydrated and condensed.

(Comparative Example 3)

A peptide-conjugated polyvinyl acetal resin and a cell culture vessel were prepared in the same manner as in Example 3 except that a linear peptide having an amino acid sequence of Gly-Arg-Gly-Asp-Ser (five amino acid residues, described as GRGDS in the table) was used, and the carboxyl group of the linker and the amino group of Gly of the peptide were dehydrated and condensed.

(Comparative Example 4)

A peptide-conjugated polyvinyl acetal resin and a cell culture vessel were prepared in the same manner as in Example 4 except that a linear peptide having an amino acid sequence of Gly-Arg-Gly-Asp-Ser (five amino acid residues, described as GRGDS in the table) was used, and the carboxyl group of the linker and the amino group of Gly of the peptide were dehydrated and condensed.

(Comparative Example 5)

A cell culture vessel was prepared in the same manner as in Example 1 except that the peptide portion was not formed.

(Example 5)

Ninety nine parts by weight of a polyvinyl alcohol with an average degree of polymerization of 1700 and a degree of saponification of 90 mol%, and 1 part by weight of acrylic acid (linker) were dissolved in 300 parts by weight of ethanol and reacted in the presence of a photoradical polymerization initiator for 20 minutes under ultraviolet irradiation to graft-copolymerize a polyvinyl alcohol with acrylic acid, thereby introducing the linker. Except for these, a peptide-conjugated polyvinyl alcohol derivative and a cell culture vessel were prepared in the same manner as in Example 1.

(Comparative Example 6)

Ninety nine parts by weight of a polyvinyl alcohol with an average degree of polymerization of 1700 and a degree of saponification of 90 mol%, and 1 part by weight of acrylic acid (linker) were dissolved in 300 parts by weight of ethanol and reacted in the presence of a photoradical polymerization initiator for 20 minutes under ultraviolet irradiation to graft-copolymerize a polyvinyl alcohol with acrylic acid, thereby introducing the linker. A peptide-conjugated polyvinyl alcohol derivative and a cell culture vessel were prepared in the same manner as in Example 1 except that a linear peptide having an amino acid sequence of Gly-Arg-Gly-Asp-Ser (five amino acid residues, described as GRGDS in the table) was used, and the carboxyl group of the linker and the amino group of Gly of the peptide were dehydrated and condensed.

(Reference Example A)

Preparation of scaffolding derived from natural product:

A Vitronectin (manufactured by Corning Incorporated) solution (1 ml) adjusted to 5 ug/ml in phosphate buffer (PBS) was added to a φ35 mm dish. A φ22 mm cover glass ("22 round No. 1" manufactured by Matsunami Glass Ind., Ltd.) was immersed therein and cured at 37°C for 1 hour, whereby scaffolding derived from a natural product in which Vitronectin (described as VTN in the table) was smoothly adsorbed on a surface was obtained.

Preparation of cell culture vessel:

A cell culture vessel was obtained in the same manner as in Example 1. Since Vitronectin is denatured when dried and its adhesive performance is significantly reduced, the cell culture vessel was immersed in a PBS solution immediately after being prepared.

(Evaluation)

(1) Presence or absence of sea-island structure

The resin films of the obtained peptide-conjugated polyvinyl acetal resin were immersed in a PBS solution for 30 minutes. The immersed resin film was observed with an atomic force microscope (AFM, "Dimension XR" manufactured by Bruker). Under measurement conditions where peak set point was set to 2 nN in QNM mode, the range of 1 $\mu$m × 1 $\mu$m was observed. The presence or absence of the sea-island structure was determined by comparing the obtained height mapping image and elastic modulus mapping image. In the table, when the sea-island structure was observed, it was described as "A", and when the sea-island structure was not observed, it was described as "B".
Fig. 2(a) is an example of an image in which the sea-island structure is determined to be observed, and Fig. 2(b) is an example of an image in which the sea-island structure is determined not to be observed.

(3) Cell culture evaluation

(3-1) Seeding and culture of iPS cells (201B7)

The following procedure was performed using the cell culture vessels obtained in Examples 1 to 4, Comparative Examples 1 to 5, and Reference Example A.
The following liquid medium and ROCK (Rho-associated kinase)-specific inhibitor were prepared.
TeSR E8 medium (manufactured by STEMCELL Technologies Inc.)

ROCK-Inhibitor (Y27632)

Phosphate buffered saline (1 mL) was added to the obtained cell culture vessel, and the mixture was allowed to stand in an incubator at 37°C for 1 hour, then the phosphate buffered saline was removed from the cell culture vessel.
To h-iPS cells 201B7 in a confluent state in a φ35 mm dish, 1 mL of a 0.5 mM ethylenediaminetetraacetic acid/phosphate buffer solution was added, and the mixture was allowed to stand at room temperature for 2 minutes. The ethylenediaminetetraacetic acid/phosphate buffer solution was removed, followed by pipetting with 1 mL of liquid medium to obtain a cell mass crushed to a size of 50 $\mu$m to 200 $\mu$m. The obtained cell mass (cell number $1.0 \times 10^5$ cells) was clamp-seeded on the cell culture vessel.

A liquid medium (1 mL), and a ROCK-specific inhibitor in an amount so as to have a final concentration of 10 $\mu$M were added to the cell culture vessel, and the cells were cultured in an incubator at 37°C and a $CO_2$ concentration of 5%. The liquid medium (1 mL) was removed every 24 hours, and 1 mL of fresh liquid medium was added to replace the medium.

(3-2) Seeding and culture of iPS cells (253G1)

The cells were seeded and cultured in the same manner as in "(3-1) Seeding and culture of iPS cells (201B7)", except that the cell culture vessels obtained in Example 5, Comparative Example 6 and Reference Example A were used, and h-iPS cells 253G1 were used.

(3-3) Extensibility of pseudopodia

Cells 24 hours after cell seeding were observed using a phase-contrast microscope (Olympus "IX73", 10 $\times$ 20 times). In the observation, images of a visual field showing the most average adhesive form in the cell culture vessel were obtained. Extensibility of pseudopodia was evaluated by determining a shape factor (SF) from the obtained images. The shape factor (SF) is a shape evaluation coefficient of a region in a plan view of a cell after culturing the cell, and is determined by the following formula.

$$ SF = 4 \times_\Pi \times (\text{Plane area of cell})/(\text{Length of outer periphery of cell})^2 \qquad \text{Formula:} $$

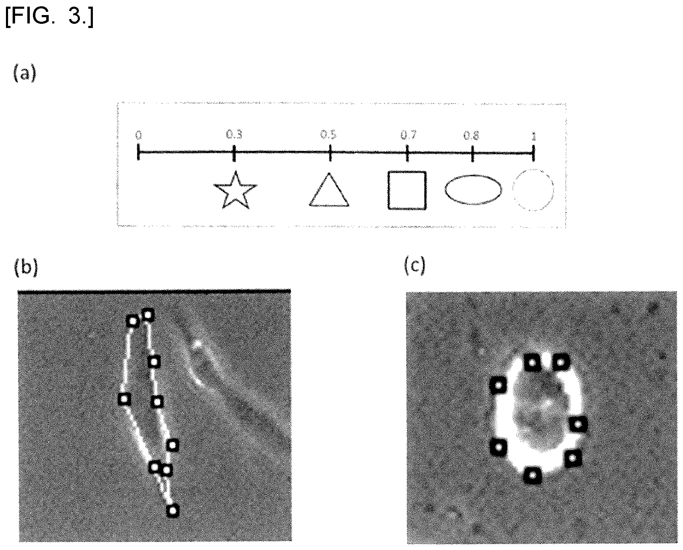

Figs. 3(a), 3(b), and 3(c) are diagrams showing a relationship between SF and a planar shape of cells. As shown in Fig. 3(a), when the SF is 1, the planar shape of the cell is circular. The smaller the SF, the farther away from the circle, which means that the pseudopodia of the cell are well extended. Fig. 3(b) is a photograph showing the planar shape of the cell when SF≈0.3, and Fig. 3(c) is a photograph showing the planar shape of the cell when SF≈1.

<Criteria for extensibility of pseudopodia>

    o: SF is 0.1 or more and 0.6 or less
    $\times$ : SF exceeds 0.6 and 1 or less

(3-4) Cell proliferation

A colonized cell mass 5 days after cell seeding was exfoliated with 1.0 mL of TryPLE Express exfoliating solution, and the number of cells was determined using a cell counter ("NucleoCounter NC-3000" manufactured by Chemometec). Next, a cell proliferation rate relative to Reference Example A was determined using the following formula. The cell proliferation rate relative to Reference Example A is compared between experiments using the same cell type (iPS cell (201B7) or iPS cell (253G1)).

Cell proliferation rate relative to Reference Example A (%) = (Number of cells in Examples or Comparative Examples)/ (Number of cells in Reference Example A) $\times$ 100

<Criteria for cell adhesion>

    A: Cell proliferation rate relative to Reference Example A is 50% or more
    B: Cell proliferation rate relative to Reference Example A is 10% or more and less than 50%
    C: Cell proliferation rate relative to Reference Example A is less than 10%

Details and results are shown in Tables 1 to 3 below.

[Table 1]

| | | | Example 1 | Example 2 | Example 3 | Example 4 |
|---|---|---|---|---|---|---|
| Polyvinyl acetal resin portion | Type | - | PVB1 | PVB2 | PVB3 | PVB4 |
| | Degree of acetalization (degree of butyralization) | Mol% | 69.3 | 62.8 | 56.2 | 44.6 5 |
| | Hydroxyl group content | Mol% | 26.7 | 23.5 | 30.1 | 41.8 |
| | Acetyl group content | Mol% | 3.0 | 2.7 | 2.7 | 2.6 |
| Linker | Carboxyl group content | Mol% | 0.9 | 1.0 | 1.0 | 1.0 |
| Peptide portion | Type | - | c-RGDfK | c-RGDfK | c-RGDfK | c-RGDfK |
| | Content | Mol% | 0.1 | 10.0 | 10.0 | 10.0 |
| Presence or absence of sea-island sLrucLure | | - | B | A | A | A |
| Cell culture evaluation | iPS cells (201B7) | Extensibility of pseudopodia | - | O | O | O | O |
| | | Cell proliferation | - | B | A | A | A |

[Table 2]

| | | | Comparative Example 1 | Comparative Example 2 | Comparative Example 3 | Comparative Example 4 | Comparative Example 5 | Reference Example A |
|---|---|---|---|---|---|---|---|---|
| Polyvinyl acetal resin portion | Type | - | PVB1 | PVB2 | PVB3 | PVB4 | PVB1 | VTN |
| | Degree of acetalization (degree of butyralization) | Mol% | 64.2 | 62.8 | 56.2 | 44.6 | 69.3 | |
| | Hydroxyl group content | Mol% | 22.1 | 23.5 | 30.1 | 41.8 | 26.7 | |
| | Acetyl group content | Mol% | 2.7 | 2.7 | 2.7 | 2.6 | 3.0 | |
| Linker | Carboxyl group content | Mol% | 1.0 | 1.0 | 1.0 | 1.0 | 1.0 | |
| Peptide portion | Type | - | RGDS | GRGDS | GRGDS | GRGDS | - | |
| | Content | Mol% | 10.0 | 10.0 | 10.0 | 10.0 | - | |
| Presence or absence of sea-island structure | | - | B | A | A | A | B | - |
| Cell culture evaluation | iPS cells (201B7) — Extensibility of pseudopodia | - | O | O | O | O | × | - |
| | iPS cells (201B7) — Cell proliferation | - | C | C | C | C | C | - |

[Table 3]

| | | | Example 5 | Comparative Example 6 | Reference Example A |
|---|---|---|---|---|---|
| Polyvinyl alcohol derivative portion | Type | - | PVA | PVA | VTN |
| | Degree of acetalization (degree of butyralization) | Mol% | 0 | 0 | |
| | Hydroxyl group content | Mol% | 90 | 90 | |
| | Acetyl group content | Mol% | 9.8 | 9.8 | |
| Linker | Carboxyl group content | Mol% | 0.1 | 0.1 | |
| Peptide portion | Type | - | c-RGDfK | GRGDS | |
| | Content | Mol% | 0.1 | 0.1 | |
| Presence or absence of sea-island structure | | - | B | B | - |
| Cell culture evaluation | iPS cells (253G1) | Extensibility of pseudopodia | - | O | O | - |
| | | Cell proliferation | - | B | C | - |

The cell culture scaffold material obtained in the examples is superior to the cell culture scaffold material obtained in the comparative examples in cell adhesion, and has excellent extensibility of pseudopodia and cell proliferation as shown in Tables 1 to 3.

EXPLANATION OF SYMBOLS

1: Cell culture vessel
2: Vessel body
2a: Surface
3: Cell culture scaffold material

**Claims**

1.   A cell culture scaffold material, comprising a peptide-conjugated polyvinyl acetal resin having a polyvinyl acetal resin portion and a peptide portion,
the peptide portion having a cyclic peptide skeleton.

2.   The cell culture scaffold material according to claim 1, wherein the peptide portion has a cell-adhesive amino acid sequence.

3.   The cell culture scaffold material according to claim 2, wherein the cell-adhesive amino acid sequence has at least an RGD sequence, a YIGSR sequence, or a PDSGR sequence.

4.   The cell culture scaffold material according to claim 2 or 3, wherein the cell-adhesive amino acid sequence has at least the RGD sequence represented by Formula (1) below:

Arg-Gly-Asp-X ...            Formula (1)

In Formula (1) above, X represents Gly, Ala, Val, Ser, Thr, Phe, Met, Pro, or Asn.

5.   The cell culture scaffold material according to any one of claims 1 to 4, wherein the cyclic peptide skeleton is composed of 4 or more and 10 or less amino acids.

6.   The cell culture scaffold material according to any one of claims 1 to 5, wherein the polyvinyl acetal resin portion and the peptide portion are bound via a linker portion.

7.  The cell culture scaffold material according to claim 6, wherein the linker portion is formed by a linker, and the linker is (meth)acrylic acid.

8.  The cell culture scaffold material according to any one of claims 1 to 7, wherein the cell culture scaffold material has a sea-island structure.

9.  A cell culture vessel, comprising a vessel body and

    the cell culture scaffold material according to any one of claims 1 to 8,
    the cell culture scaffold material being arranged on a surface of the vessel body.

**Patentansprüche**

1.  Zellkulturgerüstmaterial, umfassend ein Peptid-konjugiertes Polyvinylacetalharz mit einem Polyvinylacetalharzanteil und einem Peptidanteil,
    wobei der Peptidanteil ein cyclisches Peptidgerüst aufweist.

2.  Zellkulturgerüstmaterial nach Anspruch 1, wobei der Peptidanteil eine zelladhäsive Aminosäuresequenz aufweist.

3.  Zellkulturgerüstmaterial nach Anspruch 2, wobei die zelladhäsive Aminosäuresequenz mindestens eine RGD-Sequenz, eine YIGSR-Sequenz oder eine PDSGR-Sequenz aufweist.

4.  Zellkulturgerüstmaterial nach Anspruch 2 oder 3, wobei die zelladhäsive Aminosäuresequenz mindestens die in der untenstehenden Formel (1) dargestellte RGD-Sequenz aufweist:

    Arg-Gly-Asp-X ...          Formel (1)

    In der obigen Formel (1) steht X für Gly, Ala, Val, Ser, Thr, Phe, Met, Pro oder Asn.

5.  Zellkulturgerüstmaterial nach einem der Ansprüche 1 bis 4, wobei das cyclische Peptidgerüst aus 4 oder mehr und 10 oder weniger Aminosäuren besteht.

6.  Zellkulturgerüstmaterial nach einem der Ansprüche 1 bis 5, wobei der Polyvinylacetalharzanteil und der Peptidanteil über einen Linkeranteil verbunden sind.

7.  Zellkulturgerüstmaterial nach Anspruch 6, wobei der Linkeranteil durch einen Linker gebildet wird und der Linker (Meth)acrylsäure ist.

8.  Zellkulturgerüstmaterial nach einem der Ansprüche 1 bis 7, wobei das Zellkulturgerüstmaterial eine Meer-Insel-Struktur aufweist.

9.  Zellkulturgefäß, umfassend einen Gefäßkörper und

    das Zellkulturgerüstmaterial nach einem der Ansprüche 1 bis 8,
    wobei das Zellkulturgerüstmaterial auf einer Oberfläche des Gefäßkörpers angeordnet ist.

**Revendications**

1.  Matériau de support de culture cellulaire, comportant une résine de polyacétal de vinyle conjuguée à un peptide ayant une portion de résine de polyacétal de vinyle et une portion de peptide,
    la portion de peptide ayant un squelette de peptide cyclique.

2.  Matériau de support de culture cellulaire selon la revendication 1, dans lequel la portion de peptide a une séquence d'acides aminés d'adhérence cellulaire.

3.  Matériau de support de culture cellulaire selon la revendication 2, dans lequel la séquence d'acides aminés d'adhérence cellulaire a au moins une séquence RGD, une séquence YIGSR ou une séquence PDSGR.

4.  Matériau de support de culture cellulaire selon la revendication 2 ou 3, dans lequel la séquence d'acides aminés d'adhésion cellulaire a au moins la séquence RGD représentée par la Formule (1) ci-dessous :

Arg-Gly-Asp-X ...              Formule (1)

dans la Formule (1) ci-dessus, X représente Gly, Ala, Val, Ser, Thr, Phe, Met, Pro ou Asn.

5.  Matériau de support de culture cellulaire selon l'une quelconque des revendications 1 à 4, dans lequel le squelette de peptide cyclique est composé de 4 ou plus et de 10 ou moins acides aminés.

6.  Matériau de support de culture cellulaire selon l'une quelconque des revendications 1 à 5, dans lequel la portion de résine de polyacétal de vinyle et la portion de peptide sont liées via une portion de lieur.

7.  Matériau de support de culture cellulaire selon la revendication 6, dans lequel la portion de lieur est formée par un lieur, et
le lieur est de l'acide (méth)acrylique.

8.  Matériau de support de culture cellulaire selon l'une quelconque des revendications 1 à 7, dans lequel le matériau de support de culture cellulaire a une structure mer-îlot.

9.  Récipient de culture cellulaire, comportant un corps de récipient et

le matériau de support de culture cellulaire selon l'une quelconque des revendications 1 à 8,
le matériau de support de culture cellulaire étant disposé sur une surface du corps de récipient.

[FIG. 1.]

[FIG. 2.]

(a)                                              (b)

[FIG. 3.]

(a)

(b)

(c)

## REFERENCES CITED IN THE DESCRIPTION

**Patent documents cited in the description**

- JP 2018064542 A
- JP 2006314285 A
- WO 2007090102 A1
- JP 2017023008 A
- JP 2015070832 A

**Non-patent literature cited in the description**

- *The Journal of Cell Biology*, September 1995, vol. 130 (5), 1189-1196
- *Protein, Nucleic Acid and Enzyme*, 2000, vol. 45 (15), 2477
- *Medicina Philosophica*, 1990, vol. 9 (7), 527-535
- *Journal of Osaka Women's and Children's Hospital*, 1992, vol. 8 (1), 58-66